Europäisches Patentamt

European Patent Office

Office européen des brevets

(19)

(11) Numéro de publication : **0 132 201**
**B1**

(12) ## FASCICULE DE BREVET EUROPÉEN

(45) Date de publication du fascicule du brevet :
**22.10.86**

(21) Numéro de dépôt : **84401484.5**

(22) Date de dépôt : **12.07.84**

(51) Int. Cl.⁴ : **C 07 C 59/84**, C 07 C 51/00,
**C 07 C 51/41**

(54) **Procédé de préparation du phénylpyruvate de sodium monohydraté cristallisé.**

(30) Priorité : **18.07.83 FR 8311792**

(43) Date de publication de la demande :
**23.01.85 Bulletin 85/04**

(45) Mention de la délivrance du brevet :
**22.10.86 Bulletin 86/43**

(84) Etats contractants désignés :
**AT BE CH DE FR GB IT LI NL**

(56) Documents cités :
**JOURNAL OF THE CHEMICAL SOCIETY, CHEMICAL
COMMUNICATIONS, no. 24, 20 décembre 1978, pages
1090-1091 ; HERVE DES ABBAYES et al.: "Double
carbonylation of substituted benzyl chlorides with
cobalt carbonyl anion by phase transfer catalysis to
give arylpyruvic acids"**

(73) Titulaire : **SOCIETE FRANCAISE HOECHST Société
anonyme dite:
3, avenue du Général de Gaulle
F-92800 Puteaux (FR)**

(72) Inventeur : **Schouteeten, Alain
17 rue de Normandie
F-95460 Ezanville (FR)**
Inventeur : **Christidis, Yani
53 Boulevard de la Villette
F-75019 Paris (FR)**

(74) Mandataire : **Rinuy, Santarelli
14, avenue de la Grande Armée
F-75017 Paris (FR)**

Il est rappelé que : Dans un délai de neuf mois à compter de la date de publication de la mention de la délivrance du
brevet européen toute personne peut faire opposition au brevet européen délivré, auprès de l'Office européen des
brevets. L'opposition doit être formée par écrit et motivée. Elle n'est réputée formée qu'après paiement de la taxe
d'opposition (Art. 99(1) Convention sur le brevet européen).

## Description

La présente invention concerne un procédé de préparation de l'oxo-2 phényl-3 propionate de sodium cristallisé avec une molécule d'eau, ci-après désigné phénylpyruvate de sodium monohydraté cristallisé. Ce sel est une matière première précieuse pour préparer la phénylalanine.

Le phénylpyruvate de sodium monohydraté cristallisé est connu. Il a été obtenu, entre autres, par R. HEMMERLE lors de l'hydrolyse du cyano-3 oxo-2 phényl-3 propionate d'éthyle (Annales de Chimie et de Physique, 1917, (9), *7*, 226-276). On sait aussi le préparer par hydrolyse de la benzylidène-5 hydantoine (B. A. IVIN *et al.* Zhur. org. Khim. 1977, *13* (9), 1970-80) issue de la condensation du benzaldéhyde sur l'hydantoine (H. L. WHEELER *et al.* Amer. Chem. J., 1911, *45*, 369 ; W. J. BOYD, Biochem. J. 1935, 29, 542-5 ; The DOW CHEMICAL Co. brevet des Etats-Unis d'Amérique n° 2 861 079 ; DEGUSSA A. G., demandes de brevets européens n°s 0 037 479 et 0 037 480, demande de brevet français n° 2 485 011). L'analyse de ces documents montre que pour accéder au phénylpyruvate de sodium cristallisé avec une molécule d'eau à partir du benzaldéhyde et de l'hydantoine, en présence d'un catalyseur tel que l'acétate d'ammonium ou les alcanol amines, il est nécessaire d'effectuer cette synthèse en deux stades, avec l'isolement de la benzylidène-5 hydantoine intermédiaire. Industriellement, cette voie est donc peu économique et l'on a recherché d'autres voies de synthèse telles que la carbonylation des halogénures de benzyle (MONTEDISON S.p.A., brevet des Etats-Unis d'Amérique n° 4 351 952 ; RHONE-POULENC S.A., demande de brevet français n° 2 297 200).

Or, la demanderesse a découvert avec étonnement que l'on peut obtenir le phénylpyruvate de sodium cristallisé pur avec une molécule d'eau de solvatation avec un excellent rendement, en un seul stade, au départ du benzaldéhyde et de l'hydantoine. Le procédé selon la présente invention est d'autant plus surprenant qu'il est rapide, peu onéreux et extrêmement simple à mettre en œuvre.

Selon l'invention, ce procédé est caractérisé par le fait que l'on chauffe au reflux pendant quelques heures, une suspension aqueuse équimoléculaire de benzaldéhyde et d'hydantoine en présence d'une quantité catalytique d'éthanolamine, que l'on poursuit le reflux pendant quelques dizaines de minutes, à pH = 14, après addition dans le milieu réactionnel d'un excès de soude, que l'on acidifie ensuite la solution obtenue et refroidie à la température ambiante, à pH = 9 avec de l'acide chlorhydrique concentré, puis que l'on essore et lave par empâtage à l'eau glacée le produit cristallisé ainsi obtenu et qu'enfin on le sèche sous vide à 60 °C à poids constant.

Avantageusement, le procédé selon l'invention est effectué en chauffant 4 heures au reflux une suspension constituée d'une mole de benzaldéhyde, d'une mole d'hydantoine et de 0,1 mole d'éthanolamine dans 240 g d'eau permutée, puis en poursuivant le reflux pendant 30 minutes en présence de 1 000 cm³ de soude 5 N, en acidifiant ensuite la solution obtenue préalablement refroidie à la température ambiante, à pH = 9, avec de l'acide chlorhydrique concentré, en essorant puis en lavant par empâtage à l'eau glacée le précipité formé et, enfin, en le séchant à poids constant sous vide à 60 °C. On obtient ainsi le phénylpyruvate de sodium cristallisé pur avec une molécule d'eau de solvatation avec un excellent rendement, supérieur à 75 % de la théorie.

Ainsi que nous l'avons mentionné précédemment, le phénylpyruvate de sodium cristallisé avec une molécule d'eau, soumis à une réaction d'amination réductrice, conduit à la phénylalanine (UNITIKA Ltd, demande de brevet japonais n° 52 (77)-48 601).

L'exemple suivant illustre l'invention sans toutefois la limiter :

### Exemple 1

On chauffe 4 heures au reflux, sous agitation et sous atmosphère inerte :
— 106 g (1 mole) de benzaldéhyde,
— 100 g (1 mole) d'hydantoine,
— 6,1 g (0,1 mole) d'éthanolamine
dans 240 g d'eau permutée.

Au cours du reflux, le milieu réactionnel passe en solution puis redonne une suspension.

On introduit ensuite 1 000 cm³ de soude 5 N, soit 5 moles d'hydroxyde de sodium, puis on poursuit le reflux pendant 30 minutes. La solution obtenue est ensuite refroidie à la température ambiante, puis elle est amenée à pH = 9 par addition d'acide chlorhydrique concentré. Le produit cherché cristallise lentement ; on laisse mûrir le précipité pendant 24 heures, puis on l'essore et ensuite on le lave par empâtage avec 1 volume d'eau glacée. On obtient ainsi, après séchage à poids constant, à 60 °C, sous vide, un premier jet de 153,1 g (0,75 mole) de phénylpyruvate de sodium cristallisé avec une molécule d'eau, incolore et homogène à la chromatographie sur couche mince.

Par concentration des eaux-mères, on isole un deuxième jet de 28,5 g (0,14 mole) de phénylpyruvate de sodium cristallisé avec une molécule d'eau, incolore et homogène à la chromatographie sur couche mince.

Le rendement global s'établit à 89 % de la théorie.
Microanalyse :

C₉H₇NaO₃, H₂O

| | C % | H % | H₂O % * |
|---|---|---|---|
| calculés | 52,94 | 4,44 | 8,8 |
| trouvés | 52,8 | 4,4 | 9,3 |

* déterminé par Karl Fischer

Il va de soi, que la présente invention, n'a été décrite qu'à titre purement explicatif et nullement limitatif, et que toute modification utile, pourra y être apportée sans sortir de son cadre.

## Revendications

1. Procédé de fabrication du phénylpyruvate de sodium cristallisé monohydraté à partir du benzaldéhyde et de l'hydantoine, caractérisé par le fait que l'on fait réagir, à chaud, en milieu aqueux, en présence d'une quantité catalytique d'éthanolamine, le benzaldéhyde sur la quantité stœchiométrique d'hydantoine, que l'on traite ensuite le milieu réactionnel à chaud, à pH = 14, par un excès de soude, puis que l'on acidifie à pH = 9 avec de l'acide chlorhydrique concentré la solution ainsi obtenue et que l'on sépare le précipité formé, par des moyens connus en soi.

2. Procédé de fabrication du phénylpyruvate de sodium cristallisé monohydraté selon la revendication 1, caractérisé par le fait qu'après traitement à la soude et à l'acide chlorhydrique, on essore le précipité formé, on le lave à l'eau glacée et enfin on le sèche à poids constant sous vide à 60 °C.

## Claims

1. Process for the preparation of crystalline sodium phenylpyruvate monohydrate from benzaldehyde and hydantoin, characterized in that the benzaldehyde is reacted at raised temperature with the stoichiometric quantity of hydantoin in the presence of a catalytic quantity of ethanolamine, the reaction medium is then treated at raised temperature and with pH = 14 with excess sodium carbonate, concentrated hydrochloric acid and is then added to the solution obtained so as to obtain pH = 9, and the precipitate formed is separated out by known means.

2. Process for the preparation of crystalline sodium phenylpyruvate monohydrate according to claim 1, characterized in that after the treatment with the sodium carbonate and hydrochloric acid the precipitate is dried, washed with iced water and dried to constant weight in vaccuo at 60 °C.

## Patentansprüche

1. Verfahren zur Herstellung von kristallinem Monohydrat des Natriumsalzes von Phenylbrenztraubensäure ausgehend von Benzaldehyd und Hydantoin, dadurch gekennzeichnet, daß man Benzaldehyd in der Wärme in wässerigem Medium in Anwesenheit eines katalytischen Anteils Äthanolamin mit dem stöchiometrischen Anteil Hydantoin reagieren läßt, anschließend das Reaktionsmedium in der Wärme mit einem Überschuß an Natronlauge auf pH 14 bringt, dann die so erhaltene Lösung mit konzentrierter Salzsäure auf pH 9 ansäuert und den gebildeten Niederschlag in an sich bekannter Weise abtrennt.

2. Verfahren zur Herstellung von kristallinem Monohydrat des Natriumsalzes von Phenylbrenztraubensäure nach Anspruch 1, dadurch gekenzeichnet, daß man nach Behandlung mit Natronlauge und Salzsäure den gebildeten Niederschlag zentrifugiert, mit Eiswasser wäscht und schließlich unter Vakuum bei 60 °C auf ein konstantes Gewicht trocknet.